(19) Europäisches Patentamt

European Patent Office

Office européen des brevets

(11) **EP 1 190 764 B1**

(12) **EUROPÄISCHE PATENTSCHRIFT**

(45) Veröffentlichungstag und Bekanntmachung des Hinweises auf die Patenterteilung:
**06.10.2004 Patentblatt 2004/41**

(51) Int Cl.$^7$: **B01J 19/02**, B01J 19/12, C07C 51/58, C07C 53/48, C07C 53/50

(21) Anmeldenummer: **01130562.0**

(22) Anmeldetag: **28.11.1997**

(54) **Herstellung von Carbonsäurefluoriden**

Manufacturing of carbonxylic acid fluorides

Préparation de fluorures d'acides carboxyliques

(84) Benannte Vertragsstaaten:
**DE ES FR GB IT**

(30) Priorität: **04.12.1996 DE 19650212**

(43) Veröffentlichungstag der Anmeldung:
**27.03.2002 Patentblatt 2002/13**

(62) Dokumentnummer(n) der früheren Anmeldung(en) nach Art. 76 EPÜ:
**97952004.6 / 0 942 898**

(73) Patentinhaber: **Solvay Fluor und Derivate GmbH 30173 Hannover (DE)**

(72) Erfinder:
• **Braun, Max**
  **30900 Wedemark (DE)**
• **Eichholz, Kerstin**
  **30855 Langenhagen (DE)**
• **Rudolph, Werner**
  **30559 Hannover (DE)**

(74) Vertreter: **Fischer, Reiner et al Solvay Pharmaceuticals GmbH Hans-Böckler-Allee 20 30173 Hannover (DE)**

(56) Entgegenhaltungen:
**EP-A- 0 668 261**       **WO-A-88/04281**
**WO-A-91/09673**       **WO-A-95/09815**

Bemerkungen:
Die Akte enthält technische Angaben, die nach dem Eingang der Anmeldung eingereicht wurden und die nicht in dieser Patentschrift enthalten sind.

**Beschreibung**

Die Erfindung bezieht sich auf eine Vorrichtung, die zur Herstellung von Carbonsäurefluoriden durch photochemische Oxidation geeignet ist.

Polyhalogenierte Carbonsäurefluoride sind Zwischenprodukte in der chemischen Synthese. Sie können beispielsweise für die Herstellung wasserabstoßender bzw. ölabstoßender Verbindungen zur Anwendung bei Papier und Geweben eingesetzt werden. Sie können auch als Vorstufe für die Herstellung von oberflächenaktiven Mitteln oder im Flammschutz verwendet werden.

Die Herstellung von Perfluorcarbonsäurefluoriden kann gemäß der europäischen Patentanmeldung EP-A-260713 durch thermische Behandlung von Perfluoralkylvinylethern in Anwesenheit spezieller fluoridhaltiger Salze wie Alkalifluoride, Erdalkalifluoride, Fluormetallate, Übergangsmetallfluoride oder Oxyfluoride hergestellt werden. Für die thermische Behandlung ist eine Mindesttemperatur von 250 °C notwendig. Umwandlungsrate und Selektivität sind über den zeitlichen Verlauf der Reaktionsdurchführung nicht konstant: anfangs sind die Umwandlungsrate und die Selektivität gering.

Die internationale Patentanmeldung WO 96/29298 mit dem Titel "Verfahren zur Herstellung von Polyfluoracylzubereitungen" offenbart, daß man Polyhaloacylfluoride wie Trifluoracetylfluorid und Difluoracetylfluorid durch Oxidation von 1-Chloro-1,2,2,2-tetrafluorethan bzw. 1,1-Dichlor-2,2-difluorethan herstellen kann. Es handelt sich um ein thermisches Verfahren, das in Bezug auf Temperatur und Druck bei oder über dem kritischen Punkt durchgeführt wird. Das Verfahren ist bezüglich der Bildung des Carbonsäurefluorids nicht sehr selektiv: bei der Herstellung von Trifluoracylverbindungen entsteht Trifluoracetylfluorid in einer Menge von mindestens 60 bis 70 Mol-%, Trifluoracetylchlorid in einer Menge von 5 bis 15 Mol-%, Trifluoressigsäure in einer Menge von 10 bis 20 Mol-%, 5 bis 10 Mol-% eines Dimerisationsproduktes (Octafluordichlorbutan), und 1 bis 5 Mol-% Chlorfluorphosgen.

Die europäische Patentanmeldung EP-A-0 668261 Al offenbart die Herstellung von Polyfluorpropionylhalogeniden. Perfluorpropinylfluorid kann aus Dichlorpentafluorpropan durch Oxidation mit Sauerstoff unter Bestrahlung in einer Bestrahlungsapparatur hergestellt werden. Der Photoreaktor kann mittels Fluorpolymeren, die UV-durchlässig und HF-resistent sind, geschützt werden.

Die internationale Patentanmeldung WO 95/09815 beschreibt eine UV-Bestrahlungseinrichtung für die Behandlung von Flüssigkeiten. Die Vorrichtung soll bei der Abwasserbehandlung die sichere Abtötung von Mikroben gewährleisten. Um zu verhindern, dass Material die Wände des Reaktors belegt, wird dieser beispielsweise mit einem Schrumpfschlauch überzogen. Dieser kann beispielsweise aus PTFE® sein.

Die WO 88/04281 betrifft Verbesserungen bei Sterilisationsapparaturen. Es wird erwähnt, dass Teflon® durchlässig ist für antimikrobielle Bestrahlung mittels UV-Licht.

Die WO 91/09673 offenbart eine UV-Lampe für eine Oxidationskammer zur Abwasserbehandlung. Eine dünne Schicht aus Fluorethylenpropylen wird aufgebracht, um die Reaktoroberfläche gegen Abscheidung von Schleim zu schützen und um die Quarzglaswand mechanisch zu schützen.

Aufgabe der vorliegenden Erfindung ist es, eine Bestrahlungsapparatur anzugeben, die gegen die Einwirkung von Fluorwasserstoff geschützt ist.

Die Herstellung von Carbonsäurefluoriden der allgemeinen Formel RCFXC(O)F umfaßt die photochemische Oxidation von Verbindungen der allgemeinen Formel RCFXCHFCl als Edukt, wobei R für F oder lineares perfluoriertes C1-C9-Alkyl oder verzweigtes, perfluoriertes C1-C9-Alkyl steht und X Chlor oder Fluor bedeutet, mit Sauerstoff als Reaktant in der Gasphase. Man arbeitet also unter aktivierender Bestrahlung der gasförmigen Reaktionsmischung. Dabei kann die Bestrahlung durch Glas beliebiger Zusammensetzung (beispielsweise durch Glas innerhalb der Apparaturen, Glaseinfassungen des Strahlers) erfolgen. Beispielsweise kann man Quarzglasapparaturen einsetzen. Die Umsetzungsrate wird erhöht, wenn man in Anwesenheit eines Sensibilisators, insbesondere in Anwesenheit von elementarem Chlor als Sensibilisator, arbeitet. Dabei kann man Strahler wie Bestrahlungslampen und Leuchtstoffröhren einsetzen, die beispielsweise innerhalb eines Bereichs von 200 bis 600 nm abstrahlen. Vorteilhaft nimmt man eine aktivierende Bestrahlung mit Licht einer Wellenlänge von $\lambda \geq 280$ nm vor.

Umsatzrate, Ausbeute und Selektivität sind besonders hoch, wenn man in Anwesenheit von elementarem Chlor umsetzt und eine aktivierende Bestrahlung mit Licht einer Wellenlänge $\lambda \geq 280$ nm vornimmt. Frequenzen einer Wellenlänge unterhalb von 280 nm sind dann im wesentlichen aus dem Frequenzspektrum ausgeblendet. Dies kann man dadurch bewirken, daß man Bestrahlungslampen verwendet, die nur Licht einer Wellenlänge oberhalb oder bei 280 nm abstrahlen, und/oder man verwendet Mittel, die die entsprechenden Frequenzen aus dem abgestrahlten Licht ausblenden. Beispielsweise kann man durch Glas bestrahlen, welches nur für Licht einer Wellenlänge von 280 nm oder darüber durchlässig ist, also den kürzerwelligeren Strahlungsanteil herausfiltert. Gut geeignet dafür sind beispielsweise Borosilikat-Gläser. Geeignete Gläser enthalten beispielsweise 7 bis 13% $B_2O_3$, 70 bis 80% $SiO_2$, ferner 2 bis 7% $Al_2O_3$ und 4 bis 8% $Na_2O + K_2O$ sowie 0 bis 5% Erdalkalimetalloxide (jeweils Gew.-%). Bekannte Warenzeichen für Borosilikat-Gläser sind Duran, Pyrex und Solidex.

Zur Bestrahlung sind besonders gut Bestrahlungslampen geeignet, die nur (UV)Licht einer Wellenlänge oberhalb von oder bei 280 nm abstrahlen. Insbesondere Leuchtstoff-Röhren (z.B. von der Firma Philips) sind sehr gut geeignet. Mit derartigen Lampen kann man die Bestrahlung durch Quarzglas, aber auch durch die vorstehend beschriebenen, den kürzerwelligen Bestrahlungsanteil herausfilternde Gläser vornehmen. Voraussetzung ist natürlich, daß die verwendeten Lampen oder Röhren auch im Absorptionsbereich des elementaren Chlors emittieren. Neben den besonders gut geeigneten Leuchtstoffröhren kann man auch beispielsweise Bestrahlungslampen (z.B. Quecksilber-Mittel- oder Hochdruckstrahler) verwenden; etwaige Linien im Bereich unterhalb von 280 nm werden herausgefiltert, beispielsweise indem man durch ein Glas bestrahlt, das nur für Licht einer Wellenlänge bei oder oberhalb von 280 nm durchlässig ist. Verwendbare Gläser sind weiter oben beschrieben. Gut geeignet zur Bestrahlung sind auch Lampen, z.B. Quecksilber-Hochdrucklampen, die aufgrund eines Dotierungsmittels überwiegend oder nur im bevorzugten Wellenbereich bei oder oberhalb von 280 nm abstrahlen. Quecksilber-Hochdruckstrahler beispielsweise weisen eine recht intensive Bande im Bereich von 254 nm auf, die, wie oben beschrieben wird, beispielsweise durch Borosilikat-Glas herausgefiltert werden kann. Bei durch Metalljodide dotierten Quecksilber-Hochdruckstrahlern ist diese Linie stark unterdrückt. Überraschend ist die oft überproportionale Erhöhung der Umsatzrate bei Verwendung solcher dotierter Strahler. Besonders gut geeignet sind Quecksilber-Hochdruckstrahler, die mit Galliumjodid, insbesondere Thalliumjodid oder Cadmiumjodid dotiert sind. Auch bei Verwendung solcher dotierter Strahlungslampen filtert man vorteilhaft den kürzerwelligen Strahlungsanteil mit $\lambda$ < 280 nm heraus, beispielsweise indem man in Borosilikat-Glas arbeitet.

In Bezug auf Umsetzungstemperatur und Druck kann man derart arbeiten, daß es zu keiner Kondensation innerhalb des Photoreaktors kommt. Bei Einsatz höhersiedender Edukte kann man beispielsweise im Vakuum arbeiten. In Bezug auf die Temperatur arbeitet man vorteilhaft bei Temperaturen bis zu 200°C. Bevorzugter Temperaturbereich ist 30 bis 150°C. Wie gesagt kann man bei vermindertem Druck arbeiten, vorzugsweise beträgt der Druck mindestens 1 bar absolut. Besonders bevorzugt arbeitet man bei einem Druck von 1 bis 10 bar (abs.). Ganz besonders bevorzugt arbeitet man drucklos. Der Begriff "drucklos" bedeutet im Rahmen der vorliegenden Erfindung, daß auf die Reaktionsmischung außer dem Umgebungsdruck (d.h. etwa 1 bar), dem Förderdruck des Sauerstoffgases (bzw. des sauerstoffhaltigen Gases, man kann z.B. Luft oder Sauerstoff/-Inertgas-Gemische einsetzen) und des gegebenenfalls eingesetzten Chlors sowie dem sich gegebenenfalls ausbildenden Druck durch bei der Reaktion entstehendes Chlorwasserstoffgas kein zusätzlicher Druck einwirkt. Der Gesamtdruck im Reaktor ist dann zweckmäßig kleiner als 2 bar absolut, je nach Förderdruck sogar kleiner als 1,5 bar absolut, aber größer als der Umgebungsdruck.

Das Verfahren kann batchweise oder kontinuierlich durchgeführt werden, wobei man die Umsetzung vorteilhaft in einer Durchflußapparatur durchführt. Vorzugsweise geht man so vor, daß man kontinuierlich Ausgangsmaterial (das entsprechende Edukt, Sauerstoff bzw. ein sauerstoffenthaltendes Gas und gegebenenfalls Chlor) in die Durchflußapparatur einspeist und entsprechend der eingespeisten Menge kontinuierlich Reaktionsprodukt abzieht.

Das Molverhältnis zwischen dem Edukt und Sauerstoff kann in einem weiten Bereich schwanken, zweckmäßig setzt man jedoch mindestens 0,4 Mol Sauerstoff pro Mol Ausgangsverbindung ein. Besonders gute Ergebnisse werden erzielt, wenn das Molverhältnis zwischen der Ausgangsverbindung und dem Sauerstoff im Bereich von 1:0,4 bis 1:1, insbesondere 1:0,4 bis 1:0,6 liegt. Der Sauerstoff kann wie gesagt in Form von Luft, als $O_2$/Inertgas-Gemisch, vorzugsweise aber als reiner Sauerstoff eingesetzt werden. Sofern man die Bestrahlung in Anwesenheit von Chlor als Sensibilisator vornimmt, kann das Molverhältnis zwischen dem Edukt und dem elementarem Chlor ebenfalls in einem weiten Bereich schwanken z.B. von 1:0,01 bis 1:1. Besonders gut Ergebnisse werden erzielt, wenn das Molverhältnis zwischen dem Edukt und dem elementarem Chlor im Bereich von 1:0,05 bis 1:0,2 liegt.

In Bezug auf die Produktreinheit ist es wünschenswert, daß möglichst wenig Wasser bei der Umsetzung vorhanden ist (beispielsweise weniger als 30 ppm). Gewünschtenfalls kann man die Reaktanten in bekannter Weise von mitgeschlepptem Wasser befreien, z.B. mittels Molekularsieb.

Die durchschnittliche Verweilzeit im Reaktionsgefäß liegt vorteilhaft zwischen 0,01 und 30 Minuten, vorzugsweise zwischen 0,5 und 3,5 Minuten. Die optimale durchschnittliche Verweilzeit, die u.a. von der Art der Lampen, der Strahlungsleistung der Lampen und von geometrischen Parametern der Bestrahlungsapparatur abhängig ist, kann man durch einfache Handversuche und Analyse des Produktstroms, beispielsweise durch Gaschromatographie, ermitteln. Vorteilhaft kann es auch sein, die Reaktionsmischung beispielsweise durch geeignete Einbauten im Reaktor gut zu verwirbeln. Der Reaktionsmischung braucht kein flüssiges Kühlmittel beigefügt zu werden.

Bevorzugte Edukte, auf die das erfindungsgemäße Verfahren angewendet wird, sind solche, in welchen R für Fluor oder lineares perfluoriertes Cl-C4-Alkyl oder verzweigtes perfluoriertes Cl-C4-Alkyl steht. Insbesondere setzt man solche Edukte ein, in welchen R für Fluor, $CF_3$ oder $CF_3 CF_2$ steht. Insbesondere kann man folgende Carbonsäurefluoride herstellen:

$$CF_3CHClF + 1/2\ O_2\ \text{-->}\ CF_3C(O)F + HCl$$

$$CF_2ClCHClF + 1/2\ O_2\ \text{-->}\ CF_2ClC(O)F + HCl$$

$$CF_3CF_2CHClF + 1/2\ O_2 \longrightarrow CF_3CF_2C(O)F + HCl$$

$$CF_3CFClCHClF + 1/2\ O_2 \longrightarrow CF_3CFClC(O)F + HCl$$

$$CF_3CF_2CF_2CHClF + 1/2\ O_2 \longrightarrow CF_3CF_2CF_2C(O)F + HCl$$

$$CF_3CF_2CFClCHClF + 1/2\ O_2 \longrightarrow CF_3CF_2CFClC(O)F + HCl$$

Zum Schutz der Oberfläche der Glasapparaturen wird diese modifiziert, um sie gegen den Angriff durch das enstehende HCl-Gas und etwaig spurenmäßig entstehenden HF zu schützen. Hierzu zieht man "Strümpfe" über die zu schützenden Flächen. Als Material eignen sich poly- oder perfluorierte Kunststoffe wie Polytetrafluorethylen (PTFE), Perfluorpolypropylen (FEP), Perfluoralkoxy-Polymere (PFA) oder Polyvinylidendifluorid (PVDF) und ihre Gemische.

Das Verfahren hat gegenüber dem bekannten Verfahren zur Herstellung von Carbonsäurefluoriden den Vorteil, daß es mit hohem Umsatz und hoher Selektivität bei niedrigen Temperaturen durchgeführt werden kann. Es ist deshalb sehr effektiv. Außerdem kann es gewünschtenfalls bei niedrigem Druck durchgeführt werden.

Gegenstand der Erfindung ist eine Bestrahlungsapparatur, die im erfindungsgemäßen Verfahren verwendbar ist und speziell modifiziert ist. Bestrahlungsapparaturen weisen gewöhnlich einen Behälter zur Aufnahme einer zu bestrahlenden Flüssigkeit, eines Gases oder Gasgemisches auf sowie ein Behältnis, in welchem der Strahler angeordnet ist. Häufig weisen solche Apparate einen Schacht auf, in welchem der Strahler angeordnet ist (Tauchschachtreaktoren). Bekannt sind Bestrahlungsapparaturen aus Glas, z.B. Quarz- oder Borosilikatglas mit Glasbehältnis. Die erfindungsgemäße Bestrahlungsapparatur weist einen Behälter zur Aufnahme eines zu bestrahlenden Gases und einen Strahler auf, welcher in einem Behältnis angeordnet ist, das in Kontakt mit dem zu bestrahlenden Gas steht, wobei mindestens ein Teil der in Kontakt mit dem zu bestrahlenden Gas stehenden Oberfläche des Behältnisses eine strahlungs-durchlässige Schutzbeschichtung in Form eines Schrumpfschlauches aufweist aus strahlungsdurchlässigem (d.h. lichtdurchlässigem) polyfluoriertem oder perfluoriertem Polymer wie PTFE, FEP, PFA, PVDF oder deren Gemische. Bevorzugte Schutzbeschichtungen sind oben genannt, z.B. solche aus Polytetrafluorethylen (PTFE), FEP oder deren Gemische. Besonders bevorzugt sind erfindungsgemäße Bestrahlungsapparaturen, welche eine Schutzbeschichtung in Form eines PTFE- oder PTFE-FEP-Schrumpfschlauches aufweisen, der auf dem Glasbehältnis des Strahlers aufgezogen ist. Insbesondere handelt es sich bei den erfindungsgemäßen Bestrahlungsapparaturen um Tauchschachtreaktoren, wobei ein PTFE- oder PTFE-FEP-Schrumpfschlauch auf dem Kühlfinger des Tauchschachtes aufgebracht ist, d.h. auf derjenigen Glasoberfläche des Kühlwassermantels, welche in Kontakt mit dem zu bestrahlenden Gas oder Gasgemisch steht. Geeignete Polymere, die bei der Anwendungstemperatur klar sind und deshalb lichtdurchlässig, sind im Handel erhältlich.

Ein weiterer Gegenstand der Erfindung betrifft die Verwendung von polyfluorierten und perfluorierten Polymer-Beschichtungen in Form eines PTFE-Perfluorpropylen-Schrumpfschlauches, zum Schutz von Glasapparaturen gegen Einwirkung von Fluorwasserstoff.

Photochemisch arbeitende Verfahren, bei welchen Fluorwasserstoff als Reaktant eingesetzt oder - oft nur in Spuren - als Reaktionsprodukt ensteht, sind bekannt. Die deutsche Offenlegungsschrift DE-OS 24 18 676 offenbart die Herstellung von Trifuoracetylchlorid durch photochemische Oxidation von 1,1,1-Trifluor-2,2-Difluorethan mit Sauerstoff in Anwesenheit oder Abwesenheit von Chlor. Gasphasenverfahren zur Herstellung von halogenierten Säurechloriden offenbaren die US-Patente 5,569,782 und 5,545,298. Offenbart wird die Oxidation von Wasserstoff enthaltenden Chlorfluoralkanen in Anwesenheit von Chlor unter Bestrahlung mit bestimmten Wellenlängen des UV-Lichts ($\lambda \geq$ = 280nm, beispielsweise durch Borosilicat-Glas, bzw. die Bestrahlung in Abwesenheit von Chlor durch Quarzgas). Ein photochemisches Flüssigphasenverfahren wird in dem US-Patent 5,259,938 beschrieben. Auch gemäß jenem Verfahren wird Licht der o.a. Wellenlänge eingestrahlt. Die photochemische Herstellung von Säurefluoriden wird beispielsweise in der internationalen Patentanmeldung WO 96/29298 offenbart. Es sind neben der Herstellung von Carbonsäurederivaten auch andere Verfahren bekannt, in welchen unter photochemischer Induktion Fluorwasserstoff eingesetzt wird oder freigesetzt wird. In der Publikation "Programme and Abstracts" des "15th International Symposium on Fluorine Chemistry", The University of British Columbia, Vancouver, Canada, 2.-7. August 1997, Abstract P(2) 148 wird von Mistuo Kurumaya, Tsunetoshi Honda, und Tatsuo Nishiyama ein Verfahren zur Herstellung von kernfluorierten aromatischen Verbindungen beschrieben. Aminoaromatische Verbindungen werden in einer Lösung aus Fluorwasserstoff und Pyridin diazotiert, worauf sich eine photochemisch induzierte Fluordediazonierung anschließt. Es entstehen dann die entsprechenden kernfluorierten aromatischen Verbindungen in guter Ausbeute. Auf diese Weise wurden von den Autoren verschiedene Arten multisubstituierter fluoraromatischer Verbindungen hergestellt, welche als pharmazeutische und Flüssigkristall-Zwischenprodukte brauchbar sind. Auch bei der Photochlorierung fluorhaltiger Halogenalkane oder -alkene kann als Nebenprodukt HF entstehen.

Erfindungsgemäß eignet sich die Verwendung einer Beschichtung aus poly- und perfluorierten Polymeren, insbesondere aus Polytetrafluorethylen, Perfluorpropylen, deren Gemischen und Copolymeren in Form eines Schrumpfschlauches zum Schutz der Glasapparatur vor der Einwirkung von Fluorwasserstoff allgemein bei photochemischen Reaktionen, in welchen Fluorwasserstoff eingesetzt wird oder entsteht. Brauchbar sind beispielsweise auch Polymere, die Perfluoralkoxy-Polymere (PFA) oder Polyvinylidenfluorid oder deren Gemische aufweisen oder daraus bestehen. Schrumpfschläuche aus Copolymeren von Polytetrafluorethylen und Perfluorpropylen sind besonders gut geeignet, z.B. Schrumpfschläuche TEFLON-FEP® der Fa. Technoplast. Vorteilhaft weisen Bestrahlungsapparaturen einen Schacht auf, in welchem der Strahler angeordnet ist (Tauchschachtreaktoren). Für die Verwendung eignen sich besonders Strahlungsapparaturen, welche eine Schutzbeschichtung in Form eines Polytetrafluorethylen-Polyperfluorpropylen-Schrumpfschlauches aufweisen, welcher auf dem Glasbehältnis des Strahlers im Tauchschacht aufgezogen ist.

Polyfluorierte und perfluorierte, HF-resistente Beschichtungen oder daraus aufgebaute Vorrichtungsteile, insbesondere solche umfassend Polytetrafluorethylen und/oder Polyperfluorpropylen, sind sowohl für Gasphasenreaktionen als auch für Flüssigphasenreaktionen verwendbar. Beispielsweise kann man die Beschichtung verwenden, um Glasapparaturen im Rahmen der Herstellung von Säurefluoriden, Säurechloriden und kernfluorierten aromatischen Verbindungen oder bei Photochlorierungen wie weiter oben beschrieben gegen die Einwirkung von Fluorwasserstoff zu schützen.

Vorteil der Erfindung ist, daß auch nach monatelanger Benutzung keinerlei Verätzung durch Einwirkung von Fluorwasserstoff an der Glasapparatur festgestellt wird. Die bestrahlung ist durch die Beschichtung möglich; auch in Langzeittests wurde keine Trübung des verwendeten Schrumpfschlauches aus PTFE-Polyperfluorpropylen beobachtet.

Die folgenden Beispiele sollen die Erfindung weiter erläutern, ohne sie in ihrem Umfang einzuschränken. Man arbeitet unter Beachtung der für das Arbeiten mit molekularem Sauerstoff notwendigen Sicherheitsvorkehrungen. Bei der Verwendung von PTFE- oder anderen Beschichtungen kann es zu statischen Aufladungen kommen, die durch eine Erdung, beispielsweise ein Metallnetz, abgeleitet werden können.

Beispiele

Beispiele 1 bis 4 (Vergleichsbeispiele):

Herstellung von Trifluoracetylfluorid aus 2-Chlor-1,1,1,2-tetrafluorethan (124) mit Hg-Tauchstrahlern

Lampen: handelsübliche Tauchstrahler der Firma Heraeus Noblelight

verwendeter Tauchstrahler:

Beispiel 1: TQ 718 nicht dotiert
Beispiel 2: TQ 718 mit Cadmiumjodid dotiert
Beispiel 3: TQ 718 nicht dotiert
Beispiel 4: TQ 718 nicht dotiert

Aufbau und Durchführung

Beispiel 1: nicht dotierter Strahler, Borosilikat-Glas

In einen ca. 900 ml fassenden Tauchschacht-Photoreaktor mit Kühlfingereinsatz für Tauchlampen aus Duran 50$^R$(Wasserkühlung) wurden 101,3 g (0,74 mol) 124, 15,1 g (0,47 mol) Sauerstoff und 5,2 g (0,07 mol) Chlor eingespeist. Alle 3 Substanzen wurden vor dem Reaktor gemischt und gemeinsam gasförmig eingeleitet. Vor dem Versuch ist die Tauchlampe 15 Minuten vorgeglüht worden, da sie erst nach dieser Zeit ihre volle Strahlungsintensität erreicht. Während dieser Zeit wurde der Reaktor mit Stickstoff gespült. Die Temperatur betrug während der 30-minütigen Reaktion 80°C. In diesem Versuch wurde ein Umsatz von 85,46 % erzielt, mit einer Selektivität bezogen auf entstandenes Trifluoracetylfluorid von 99,90%. Im Abgas wurden Spuren von TFAC nachgewiesen.

Analytik

Das Reaktorabgas wurde mittels GC untersucht, die Identifikation der Substanzen erfolgte mit GC-MS.

Beispiel 2: CdI-dotierter Strahler, Borosilikat-Glas

| Durchführung | wie Beispiel 1 |
|---|---|
| Ansatz | 105,4 g (0,77 mol) 124, 14,4 g (0,45 mol) Sauerstoff, 5,1 g (0,07 mol) Chlor |
| Reaktionstemperatur | 90°C |
| Umsatz | 93,09% |
| Selektivität | 98,03% |

Aufbau und Durchführung

Beispiel 3: nicht dotierter Strahler, Quarz-Glas

In einen ca. 900 ml fassenden Tauchschacht-Photoreaktor mit Kühlfingereinsatz für Tauchlampen aus Quarz (Wasserkühlung) wurden 69,2 g (0,51 mol) 124, 10,0 g (0,31 mol) Sauerstoff und 3,2 g (0,05 mol) Chlor eingespeist. Alle 3 Substanzen wurden vor dem Reaktor gemischt und gemeinsam gasförmig eingeleitet. Vor dem Versuch ist die Tauchlampe 15 Minuten vorgeglüht worden, da sie erst nach dieser Zeit ihre volle Strahlungsintensität erreicht. Während dieser Zeit wurde der Reaktor mit Stickstoff gespült. Die Temperatur betrug während der 20-minütigen Reaktion 115°C. In diesem Versuch wurde ein Umsatz von 97,75% erzielt, mit einer Selektivität bezogen auf entstandenes Trifluoracetylfluorid von 91,38%. Im Abgas wurden Spuren von Trifluoracetylchlorid nachgewiesen.

Beispiel 4: nicht dotierter Strahler, Quarzglas, chlorfrei

| Durchführung | wie Beispiel 3, Tauchschacht-Photoreaktor mit Kühlfingereinsatz aus Quarz |
|---|---|
| Ansatz | 109,8 g (0,80 mol) 124, 15,2 g (0,48 mol) Sauerstoff ohne Chlor |
| Reaktionstemperatur | 135°C |
| Umsatz | 92,13% |
| Selektivität | 90,85% |

Beispiel 5 (Vergleichsbeispiel):

Herstellung von Trifluoracetylfluroid aus 2-Chlor-1,1,1,2-tetrafluorethan mit Leuchtstofflampen als Strahlungsquelle

| Ansatz | 118,07 g 124 = 0,91 mol |
|---|---|
| | 19,84 g $O_2$ = 0,62 mol |
| | 9,93 g $Cl_2$ = 0,14 mol |
| Lampen | 3 x 40 Watt UV Lampen von Philips |
| | Typ: Cleo Performance R-UVA 40 W |
| | Länge: 600 mm |

Aufbau und Durchführung

In einen 4,3 Liter fassenden Photoreaktor ( ⌀ 100 mm, Wandstärke 2 mm) aus Duran 50 wurde eine Mischung aus 118,07 g (0,91 mol) 124, 19,84 g (0,62 mol) Sauerstoff und 9,93 g (0,14 mol) Chlor gasförmig eingeleitet.
Die Reaktortemperatur betrug während der 30-minütigen Reaktion 41 bis 46°C. Die Bestrahlung erfolgte mit 3 handelsüblichen UV Leuchtstofflampen der Firma Philips, die einseitig mit einer Reflektorschicht ausgestattet sind, um die Strahlungsausbeute zu erhöhen (Anordnung der Lampen zylindrisch um den Reaktor).
Der Umsatz der Reaktion betrug 65,17%, die Selektivität, bezogen auf entstandenes TFAF, lag bei 99,88%. Im GC konnten ebenfalls Spuren von Trifluoracetylchlorid nachgewiesen werden.

Analytik

Die Probenanalyse des Reaktorabgases erfolgte mittels GC, die Identifikation der Substanzen mittels GC-MS.

Beispiel 6 (Vergleichsbeispiel):

Herstellung von Chlordifluoracetylfluorid aus 1,2-Dichlor-1,1,2-trifluorethan (123a) in Duranglas

| Ansatz | 80,10 g 123a = 0,53 mol |
|---|---|
| | 10,60 g $O_2$ = 0,33 mol |
| | 3,90 g $Cl_2$ = 0,06 mol |

Aufbau und Durchführung

Durch einen 4,3 Liter fassenden Photoreaktor aus Duran 50 (Reaktoraufbau wie in Beispiel 5) werden Sauerstoff, Chlor und 123a geleitet. Sauerstoff und Chlor werden vorgemischt und nach dem auf 100°C temperierten Vorverdampfer dem 123a zugeführt. Die Dosierung des 123a in den Vorverdampfer erfolgt über eine Membranpumpe mit kühlbarem Kopf.
Die Bestrahlung erfolgt mit handelsüblichen UV Leuchtstofflampen der Firma Philips, die einseitig mit einer Reflektorschicht ausgestattet ist, um die Strahlungsausbeute zu erhöhen. Die genaue Bezeichnung ist Cleo Performance R-UVA 40 W. Die Nennleistung der Lampen beträgt 40 Watt.

Versuchsdauer 15 Minuten

123a wird verdampft und mit den anderen Reaktionskomponenten gemischt durch den Reaktor geleitet. Hinter dem Reaktor befinden sich zwei Kühlfallen mit einer Methanol/$CO_2$-Mischung, um das entstandene Chlordifluoracetylfluorid aufzufangen.
Die Reaktortemperatur betrug dabei bis zu 46°C, bestrahlt wurde mit 2 x 40 Watt.
Der Umsatz betrug 30,66%, die Selektivität bezogen auf entstandenes CDFAF lag bei 93,41%.

Analytik

Die Proben wurden mittels Gaschromatographie untersucht, die Identifikation der Substanzen erfolgte mittels GC-MS.

Beispiel 7:

Herstellung von Trifluoracetylfluorid aus R 124 mit modifiziertem Reaktor

Apparatur:

Eingesetzt wurde ein Photoreaktor mit 900 ml Reaktorvolumen. Er weist einen Tauchschacht mit wassergekühltem Kühlfinger zur Aufnahme des Strahlers auf. Der Kühlfinger des Tauchschachtes aus Duran 50 Glas war auf der in Kontakt mit dem zu bestrahlenden Gas stehenden Oberfläche mit einem Schrumpfschlauch TEFLON-FEP[R] der Firma Technoplast überzogen. Die eingesetzte Lampe hatte eine Leistung von 700 W (TQ 718, Heraeus nicht dotierter Strahler).

Durchführung:

Eine Mischung von 51,3 g (0,37 mol) 124, 6 g (0,19 mol) Sauerstoff und ca. 2,5 g (0,03 mol) Chlor wurde während einer Zeitdauer von 15 Minuten durch den Reaktor geleitet (Innentemperatur: 85°C). Eine Veränderung des Schrumpfschlauches (Trübung, Gelbfärbung) wurde nicht beobachtet. Der Umsatz betrug 94,1%, die Selektivität 99,6%.

Auch bei einem Langzeittest war keine Veränderung des Schrumpfschlauches zu beobachten.

Beispiel 8:

Test der Eignung eines Schrumpfschlauches aus Polytetrafluorethylen (PTFE) und Perfluoralkoxy-Polymeren (PFA)

Durchführung:

Ein PTFE-PFA-Schrumpfschlauch, Hersteller DuPont, wurde auf einen Tauchschacht aus Duran 50® aufgezogen und 2 Tage lang mit einem Heraeus-Strahler bestrahlt. Es wurde keine Eintrübung beobachtet, sondern der Schlauch blieb klar. Dies belegt, daß auch PTFE-PFA-Polymere sehr gut für die Herstellung von Betrahlungsappraturen bzw. Schrumpfschläuchen zur Anwendung in Bestrahlungsverfahren brauchbar sind (trotz des Sauerstoffgehaltes im Polymer).

**Patentansprüche**

1. Bestrahlungsapparatur mit einem Behälter zur Aufnahme eines zu bestrahlenden Gases und einem Strahler, der in einem Behältnis aus Glas angeordnet ist, das in Kontakt mit dem zu bestrahlenden Gas steht, **gekennzeichnet durch** eine strahlungsdurchlässige Schutzbeschichtung auf Basis von strahlungsdurchlässigen polyfluorierten oder perfluorierten Polymeren mindestens auf einen Teil der in Kontakt mit dem zu bestrahlenden Gas stehenden Oberfläche des Behältnisses, wobei die Schutzbeschichtung ein Schrumpfschlauch ist, der auf dem Glasbehältnis des Strahlers aufgezogen ist, wobei der Strahler überwiegend oder nur Licht einer Wellenlänge $\lambda \geq 280$ nm abstrahlt, oder wobei der kürzerwellige Strahlungsanteil mit $\lambda < 280$ nm herausgefiltert wird, oder beides.

2. Bestrahlungsapparatur nach Anspruch 1, **dadurch gekennzeichnet, daß** die Schutzbeschichtung oder das Behältnis aus PTFE (Polytetrafluorethylen), Polyfluorpropylen oder deren Gemischen besteht.

3. Verwendung einer für Licht einer Wellenlänge von $\lambda \geq 280$ nm strahlungsdurchlässigen Beschichtung in Form eines Schrumpfschlauches aus polyfluorierten oder perfluorierten Polymeren zum Schutz von Glasapparaturen gegen die Einwirkung von Fluorwasserstoff in photochemischen Reaktionen, in welchen Fluorwasserstoff eingesetzt wird oder entsteht.

4. Verwendung nach Anspruch 3, **dadurch gekennzeichnet, daß** die Beschichtung aus Polytetrafluorethylen, Perfluorpropylen oder deren Gemischen oder Copolymeren besteht.

5. Verwendung nach Anspruch 3 oder 4 im Rahmen der photochemischen Oxidation Wasserstoff enthaltender Halogenkohlenstoffverbindungen, der photochemisch induzierten Fluordediazonierung oder der Photochlorierung.

**Claims**

1. An irradiation apparatus comprising a container for receiving a gas to be irradiated and a radiator located in a receptacle of glass which is in contact with the gas to be irradiated, **characterised by** a radiation-transmissive protective coating based on radiation-transmissive polyfluorinated or perfluorinated polymers at least on part of the surface of the receptacle in contact with the gas to be irradiated, the protective coating being a heat-shrinkable sleeve which is pulled on to the glass receptacle of the radiator, the radiator predominantly or only emitting light of a wavelength of $\lambda \geq 280$ nm, or the shorter-wave radiation portion of $\lambda < 280$ nm being filtered out, or both.

2. An irradiation apparatus according to Claim 1, **characterised in that** the protective coating or the receptacle consists of PTFE (polytetrafluoroethylene), polyfluoropropylene or mixtures thereof.

3. The use of a coating which is transmissive to light of a wavelength of $\lambda \geq 280$ nm in the form of a heat-shrinkable sleeve of polyfluorinated or perfluorinated polymers to protect glass apparatus from the action of hydrogen fluoride in photochemical reactions in which hydrogen fluoride is used or produced.

4. The use according to Claim 3, **characterised in that** the coating consists of polytetrafluoroethylene, perfluoropropylene or mixtures or copolymers thereof.

5. The use according to Claim 3 or 4 in the context of the photochemical oxidation of hydrogen-containing halocarbon compounds, photochemically induced fluorodediazonation or photochlorination.

**Revendications**

1. Appareil d'irradiation comportant un conteneur destiné à recevoir un gaz à irradier, et un émetteur de rayonnement qui est disposé dans une enveloppe de verre, qui se trouve en contact avec le gaz à irradier, **caractérisé par** un revêtement protecteur perméable au rayonnement, à base de polymères polyfluorés ou perfluorés perméables aux rayonnements, sur au moins une partie de la surface de l'enveloppe qui se trouve en contact avec le gaz à irradier, dans lequel le revêtement protecteur est une gaine thermorétractable qui est tendue sur l'enveloppe de verre de l'émetteur de rayonnement, dans lequel l'émetteur de rayonnement émet essentiellement ou uniquement de la lumière d'une longueur d'onde $\lambda \geq 280$ nm, ou dans lequel la fraction du rayonnement de plus petite longueur d'onde est filtrée avec $\lambda < 280$ nm ou les deux.

2. Appareil d'irradiation selon la revendication 1, **caractérisé en ce que** le revêtement protecteur ou l'enveloppe se compose de PTFE (polytétrafluoroéthylène), de polyfluoropropylène ou de leurs mélanges.

3. Utilisation d'un revêtement perméable au rayonnement pour une lumière d'une longueur d'onde $\lambda \geq 280$ nm sous la forme d'une gaine thermorétractable à base de polymères polyfluorés ou perfluorés afin de protéger des appareils de verre contre l'action du fluorure d'hydrogène dans des réactions photochimiques, dans lesquelles on utilise ou il se forme du fluorure d'hydrogène.

4. Utilisation selon la revendication 3, **caractérisée en ce que** le revêtement se compose de polytétrafluoroéthylène, de perfluoropropylène ou de leurs mélanges ou copolymères.

5. Utilisation selon la revendication 3 ou 4 dans le cadre de l'oxydation photochimique de composés carbonés halogénés contenant de l'hydrogène, de la dédiazonation du fluor induite par photochimie ou de la photochloration.